# EUROPÄISCHE PATENTSCHRIFT

(11) **EP 0 597 287 B1**
(45) Veröffentlichungstag und Bekanntmachung des Hinweises auf die Patenterteilung: **10.04.1996**
(21) Anmeldenummer: 93116939.5
(22) Anmeldetag: 20.10.1993
(51) Int. Cl.: C07C 271/30, C07C 225/34, C07C 50/18, C09B 1/18

(54) **Verfahren zur Herstellung von Anthrachinonen**
Process for the preparation of anthraquinones
Procédé de préparation d'anthraquinone

(30) Priorität: 11.11.1992 DE 4238045
(43) Veröffentlichungstag der Anmeldung: 18.05.1994
(73) Patentinhaber: BASF Aktiengesellschaft, D-67063 Ludwigshafen (DE)
(72) Erfinder: Ebel, Klaus, Dr., D-6700 Ludwigshafen (DE); Schroeder, Juergen, Dr., D-6806 Viernheim (DE)

(56) Entgegenhaltungen:
- DE-A- 2 437 220
- DE-B- 2 460 922
- GB-A- 2 190 080
- TETRAHEDRON LETTERS, Bd.28, Nr.39, 1987 Seiten 4529 - 4532 M. CHIGR ET AL

## Beschreibung

Die vorliegende Erfindung betrifft ein Verfahren zur Herstellung von Anthrachinonen durch Diels-Alder-Reaktion von N-Butadienylcarbamidsäureestern mit 1,4-Naphthochinonen bzw. mit 1,4-Benzochinon, Oxidation und gegebenenfalls Abspaltung der Schutzgruppe.

Aus der GB-A-1 370 413 ist eine zweistufige Synthese für 1-Aminoanthrachinon bekannt, bei der Anthrachinon in Gegenwart von Quecksilbersulfat mit Oleum zu Anthrachinon-1-sulfonsäure sulfoniert und in einer zweiten Stufe mit Ammoniak zum 1-Amino-anthrachinon umgesetzt wurde.

Aus der GB-A-1 351 047 ist die Nitrierung von Anthrachinon zu 1-Nitroanthrachinon und die anschließende Reduktion zu 1-Aminoanthrachinon bekannt.

Diese Verfahren haben den Nachteil, daß bei der Synthese von 1-Aminoanthrachinon große Salzmengen anfallen.

Aus Tetrahedron Letters Band 28, 4529 bis 4532 (1987) ist die Diels-Alder-Reaktion von 1,4-Naphthochinonen mit N-Butadienylcarbamidsäurebenzylester bekannt, wobei die Oxidation zu 1-Benzyloxycarbonylaminoanthrachinon mit dem fünffachen Überschuß an aktiviertem Braunstein durchgeführt und die Schutzgruppe mit Bromwasserstoffsäure/Essigsäure zu den entsprechenden 1-Aminoanthrachinonen abgespalten wird.

Nachteilig bei diesem Verfahren ist die aufwendige Oxidation. Die zur Oxidation von Tetrahydroanthrachinonen üblichen Verfahren aus Houben-Weyl, Methoden der organischen Chemie, Band 7/3c, Seite 23 bis 31, wie Dehydrierung in der Schmelze durch Zugabe von Kupferpulver, mit Luft in Gegenwart von Alkalimetallhydroxiden versagen.

Der vorliegenden Erfindung lag daher die Aufgabe zugrunde, den zuvor genannten Nachteilen abzuhelfen.

Demgemäß wurde ein neues und verbessertes Verfahren zur Herstellung von Anthrachinonen der allgemeinen Formel I 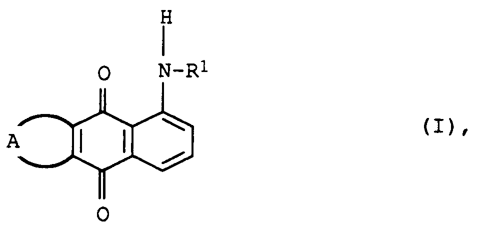
in der
- R¹: Wasserstoff oder 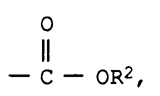
- A: 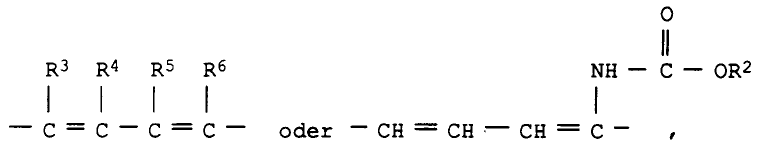
- R²: C₁- bis C₈-Alkyl, C₇- bis C₂₀-Phenylalkyl und
- R³, R⁴, R⁵, R⁶: unabhängig voneinander Wasserstoff, C₁- bis C₈-Alkyl, C₇- bis C₂₀-Phenylalkyl, C₁- bis C₈-Alkoxy, C₁- bis C₈-Halogenalkyl, Nitro, Cyano, Halogen, Amino, C₁- bis C₈-Alkyl carbonyl, Benzoyl, N-C₁- bis C₈-Alkylphenylamino, C₁- bis C₈-Alkylsulfonyl, Phenylsulfonyl, Aminosulfonyl, Aminocarbonyl und Nitrobenzyl
bedeuten, dadurch gekennzeichnet, daß man N-Butadienylcarbamidsäureester der allgemeinen Formel II 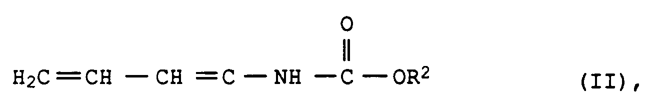
in der R² die obengenannten Bedeutungen hat,
a) für den Fall, daß A 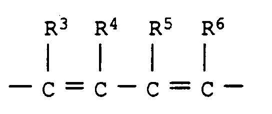 bedeutet, mit 1,4-Naphthochinonen der allgemeinen Formel III 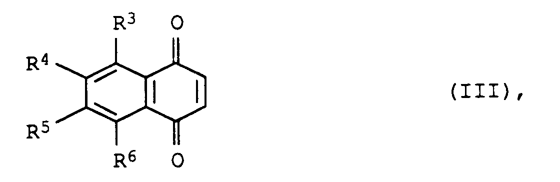 in der die Substituenten R³ bis R⁶ die obengenannten Bedeutungen haben, oder
b) für den Fall, daß A 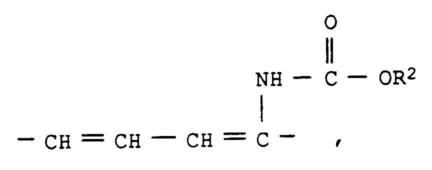 bedeutet, mit 1,4-Benzochinon
bei Temperaturen von 0 bis 150°C, und anschließend die entstandenen Carbamoylhydroanthrachinone in einem tertiären Amin mit sauerstoffhaltigen Gasen in Gegenwart von Kupfer-salzen umsetzt, und gegebenenfalls die entstandenen Carbamoylanthrachinone mit Hydroxidlösungen bei Temperaturen von 0 bis 150°C behandelt.

Das erfindungsgemäße Verfahren läßt sich wie folgt durchführen:

### - Herstellung der 1-Carbamoyl-1,4,4a,9a-tetrahydroanthrachinone bzw. der 1,5/1,8-Biscarbamoyl-1,4,4a,5,8,8a,9a,10a-octahydroanthrachinone

Man kann das Chinon (1,4-Naphthochinon III oder 1,4- Benzochinon) und den N-Butadienylcarbamidsäureester II in einem inerten Lösungsmittel vorlegen und bei 0 bis 150°C, bevorzugt 10 bis 100°C, besonders bevorzugt 20 bis 70°C und Drücken von 0,01 bis 10 bar, bevorzugt 0,1 bis 5 bar, besonders bevorzugt bei Normaldruck (Atmosphärendruck) umsetzen.

Das Molverhältnis von den N-Butadienylcarbamidsäureestern II zu den 1,4-Naphthochinone III beträgt 0,5 : 1 bis 2,5 : 1, bevorzugt 0,8 : 1 bis 1,5 : 1, besonders bevorzugt 0,9 : 1 bis 1,1 : 1.

Das Molverhältnis von den N-Butadienylcarbamidsäureestern II zum 1,4-Benzochinon beträgt 1,5 : 1 bis 4 : 1, bevorzugt 1,8 : 1 bis 3 : 1, besonders bevorzugt 1,9 : 1 bis 2,1 : 1.

Als inerte Lösungsmittel eignen sich beispielsweise C₄- bis C₃₀-Kohlenwasserstoffe wie Butane, Pentane, Hexane, Cyclohexan, Benzol, Toluol, chlorierte Kohlenwasserstoffe wie Methylenchlorid, Chloroform, Trichlorethylen und Pyridinderivate wie Pyridin, Picoline (2-, 3- und 4-), Lutidine (2,4-, 2,6-, 3,4- und 3,5-) bevorzugt Toluol, Chloroform und Pyridin.

### - Herstellung der 1-Carbamoylanthrachinone bzw. der 1,5/1,8-Bis-carbamoylanthrachinone

1-Carbamoyl-1,4,4a,9a-tetrahydroanthrachinone bzw. 1,5/1,8-Bis-carbamoyl-1,4,4a,5,8,8a,9a,10a-octahydroanthrachinone lassen sich bei Temperaturen von 0 und 150°C, bevorzugt 10 bis 100°C, besonders bevorzugt 20 und 50°C, in einem tertiären Amin, in Gegenwart von Kupfer-salzen, mit sauerstoffhaltigen Gasen, beispielsweise durch eingasen, oxidieren.

Kupfer-salze werden in der Regel im Molverhältnis von 0,001 : 1 bis 0,5 : 1, bevorzugt 0,05 : 1 bis 0,2 : 1 zum 1-Carbamoyl-1,4,4a,9a- tetrahydroanthrachinon bzw. 1,5/1,8-Biscarbamoyl-1,4,4a,5,8,8a,9a,10a-octahydroanthrachinon eingesetzt.

Als sauerstoffhaltige Gase eignen sich beispielsweise Sauerstoffgas oder bevorzugt der Sauerstoff der Umgebungsluft.

Die beiden zuvor beschriebenen Schritte können bevorzugt als Eintopfreaktion durchgeführt werden, indem man die 1,4-Naphthochinone III bzw. das 1,4-Benzochinon mit N-Butadienylcarbamidsäureestern II in den oben angegebenen Molverhältnissen bei den oben genannten Temperaturen in einem tertiären Amin, in Gegenwart von Kupfer-salzen in den oben genannten Molverhältnissen und einem sauerstoffhaltigem Gas, umsetzt.

Als tertiäre Amine eignen sich beispielsweise aliphatische Amine wie Triethylamin, Tripropylamin, Tetramethylethylendiamin oder oder aromatische Amine wie Pyridin, Picoline (2-, 3- und 4-), Lutidine (2,4-, 2,6-, 3,4- und 3,5-), bevorzugt Pyridin.

Als Kupfer-salze eignen sich beispielsweise Kupfer-I-chlorid, Kupfer-II-chlorid, Kupfer-I-bromid und Kupfer-II-acetat, bevorzugt Kupfer-I-chlorid.

### - Herstellung der 1-Aminoanthrachinone der Formel I

Die Schutzgruppe in den 1-Carbamoylanthrachinonen bzw. in den 1,5/1,8-Biscarbamoylanthrachinonen läßt sich bei Temperaturen zwischen 0 und 150°C, vorzugsweise zwischen 50 und 100°C, alkalisch, beispielsweise in wäßrig-alkoholischer Hydroxidlösung abspalten.

Als alkalische Hydroxidlösung eignen sich beispielsweise wäßrigalkoholische Lösungen von Alkali- und/oder Erdalkalihydrooxiden wie Natriumhydroxid und Kaliumhydroxid in C₁- bis C₈-Alkanolen, bevorzugt C₁- bis C₄-Alkanolen wie Methanol, Ethanol, n-Propanol und iso-Propanol.

N-Butadienylcarbamidsäureester können nach Organic Synthesis, Band 59, Seite 1 bis 9 (1979) hergestellt werden.

Das Zwischenglied A und die Substituenten R¹, R², R³, R⁴, R⁵ und R⁶ in den Verbindungen I, II und III unabhängig voneinander haben folgende Bedeutungen:
- R¹: - Wasserstoff oder 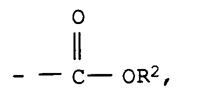
- A: 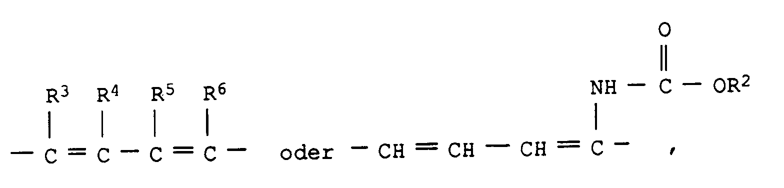
- R²,R³,R⁴,R⁵,R⁶: - C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₇- bis C₂₀-Phenylalkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- R³,R⁴,R⁵,R⁶: - Wasserstoff
- C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, tert.-Amyloxy, n-Hexoxy, iso-Hexoxy, n-Heptoxy, iso-Heptoxy, n-Octoxy und iso-Octoxy, bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy, besonders bevorzugt Methoxy und Ethoxy,
- C₁- bis C₈-Halogenalkyl, bevorzugt C₁- bis C₄-Halogenalkyl, besonders bevorzugt C₁- bis C₄-Fluor-, Chlor- und/oder Bromalkyl wie Fluormethyl, Difluormethyl, Trifluormethyl, Chlormethyl, Dichlormethyl, Trichlormethyl, Brommethyl, Dibrommethyl und Tribrommethyl,
- Nitro,
- Cyano,
- Halogen wie Fluor, Chlor, Brom und Jod, bevorzugt Fluor, Chlor und Brom, besonders bevorzugt Chlor und Brom,
- Amino,
- C₁- bis C₈-Alkylcarbonyl wie Methylcarbonyl, Ethylcarbonyl, n-Propylcarbonyl, iso-Propylcarbonyl, n-Butylcarbonyl, iso-Butylcarbonyl, sec.-Butylcarbonyl, tert.-Butylcarbonyl, n-Pentylcarbonyl, iso-Pentylcarbonyl, tert.-Amylcarbonyl, n-Hexylcarbonyl, iso-Hexylcarbonyl, n-Heptylcarbonyl, iso-Heptylcarbonyl, n-Octylcarbonyl und iso-Octylcarbonyl, bevorzugt C₁- bis C₄-Alkylcarbonyl wie Methylcarbonyl, Ethylcarbonyl, n-Propylcarbonyl, iso-Propylcarbonyl, n-Butylcarbonyl, iso-Butylcarbonyl, sec.-Butylcarbonyl und tert.-Butylcarbonyl, besonders bevorzugt Methylcarbonyl und Ethylcarbonyl,
- Benzoyl,
- N-C₁- bis C₈-Alkylphenylamino wie Methylphenylamino, Ethylphenylamino, n-Propylphenylamino, iso-Propylphenylamino, n-Butylphenylamino, iso-Butylphenylamino, sec.-Butylphenylamino, tert.-Butylphenylamino, n-Pentylphenylamino, iso-Pentylphenylamino, tert.-Amylphenylamino, n-Hexylphenylamino, iso-Hexylphenylamino, n-Heptylphenylamino, iso-Heptylphenylamino, n-Octylphenylamino und iso-Octylphenylamino, bevorzugt C₁- bis C₄-Alkylphenylamino wie Methylphenylamino, Ethylphenylamino, n-Propylphenylamino, iso-Propylphenylamino, n-Butylphenylamino, iso-Butylphenylamino, sec.-Butylphenylamino und tert.-Butylphenylamino, besonders bevorzugt Methylphenylamino und Ethylphenylamino,
- C₁- bis C₈-Alkylsulfonyl wie Methylsulfonyl, Ethylsulfonyl, n-Propylsulfonyl, iso-Propylsulfonyl, n-Butylsulfonyl, iso-Butylsulfonyl, sec.-Butylsulfonyl, tert.-Butylsulfonyl, n-Pentylsulfonyl, iso-Pentylsulfonyl, tert.-Amylsulfonyl, n-Hexylsulfonyl, iso-Hexylsulfonyl, n-Heptylsulfonyl, iso-Heptylsulfonyl, n-Octylsulfonyl und iso-Octylsulfonyl, bevorzugt C₁- bis C₄-Alkylsulfonyl wie Methylsulfonyl, Ethylsulfonyl, n-Propylsulfonyl, iso-Propylsulfonyl, n-Butylsulfonyl, iso-Butylsulfonyl, sec.-Butylsulfonyl und tert.-Butylsulfonyl, besonders bevorzugt Methylsulfonyl und Ethylsulfonyl,
- Phenylsulfonyl,
- Aminosulfonyl, - Aminocarbonyl und
- Nitrobenzyl.

1-Aminoanthrachinone sind wertvolle Zwischenprodukte für die Herstellung von Farbstoffen (Ullmanns Encyklopädie der technischen Chemie, 4. Auflage, Band 7, Seite 586 bis 646).

### Beispiele

### Beispiel 1

3,16 g 1,4-Naphthochinon, 4,48 g N-Butadienylcarbamidsäurebenzylester und 50 mg Hydrochinon wurden in 100 ml Toluol gelöst und 8 Stunden bei 25°C gerührt. Anschließend wurde die Lösung i. Vak. eingeengt. Der Rückstand wurde in Chloroform aufgenommen und über Kieselgel filtriert. Das Filtrat wurde i. Vak. eingeengt. Man erhielt 7,12 g 1-Benzyloxycarbonylamino-1,4,4a,9a-tetrahydroanthrachinon (93 %ig; 92 % der Theorie).

### Beispiel 2

0,47 g 1-Benzyloxycarbonylamino-1,4,4a,9-tetrahydroanthrachinon, 6 mg Kupfer-(I)-chlorid und 10 ml Pyridin wurden unter einem schwachen Luftstrom 30 Stunden bei 25°C gerührt. Anschließend wurde die Lösung i. Vak. eingeengt. Der Rückstand wurde in Chloroform aufgenommen und mit 2N Salzsäure extrahiert. Die organische Phase wurde i. Vak. eingeengt. Man erhielt 0,41 g 1-Benzyloxycarbonylaminoanthrachinon (86 %ig; 76 % der Theorie).

### Beispiel 3

0,50 g 1-Benzoyloxycarbonylaminoanthrachinon, 25 ml Ethanol und 25 ml 50 %ige wäßrige Natronlauge wurden 6 Stunden unter Rückfluß erhitzt. Anschließend wurde die Lösung i. Vak. eingeengt. Der Rückstand wurde in Chloroform/Wasser aufgenommen, die Phasen wurden getrennt und die organische Phase wurde zweimal mit Wasser gewaschen. Die organische Phase wurde i. Vak. eingeengt. Man erhielt 0,30 g 1-Aminoanthrachinon (99 %ig; 95 % der Theorie).

### Beispiel 4

0,79 g 1,4-Naphthochinon, 1,07 g N-Butadienylcarbamidsäurebenzylester, 25 mg Kupfer-(I)-chlorid und 25 ml Pyridin wurden unter einem schwachen Luftstrom 30 Stunden bei 25°C gerührt. Anschließend wurde die Lösung i. Vak. eingeengt. Der Rückstand wurde in Chloroform aufgenommen und mit 2N Salzsäure extrahiert. Die organische Phase wurde i. Vak. eingeengt. Man erhielt 1,60 g 1-Benzyloxycarbonylaminoanthrachinon (83 %ig; 75 % der Theorie).

### Beispiel 5

0,50 g 1-Benzyloxycarbonylamino-1,4,4a,9a-tetrahydroanthrachinon wurden in 100 ml 5 %iger ethanolischer Kaliumhydroxidlösung gelöst. Durch die Lösung wurde 6 Stunden bei 25°C Luft hindurchgeleitet. Anschließend wurde die Lösung i. Vak. eingeengt. Der Rückstand wurde in Chloroform/Wasser aufgenommen, die Phasen wurden getrennt und die organische Phase wurde zweimal mit Wasser gewaschen. Die organische Phase wurde i. Vak. eingeengt. Man erhielt ausschließlich unsubstituiertes Anthrachinon.

### Beispiel 6

0,54 g p-Benzochinon, 2,03 g N-Butadienylcarbamidsäurebenzylester, 99 mg Kupfer-(I)-chlorid und 50 ml Pyridin wurden unter einem schwachen Luftstrom 44 Stunden bei 25°C gerührt. Anschließend wurde die Lösung i. Vak. eingeengt. Der Rückstand wurde in Toluol aufgenommen und mit 1N Salzsäure extrahiert. Die organische Phase wurde i. Vak. eingeengt. Man erhielt 2,30 g 1,8- und 1,5 -Bis-(benzyloxycarbonylamino)-anthrachinon (98 %ig; 89 % der Theorie).

## Patentansprüche

1. Verfahren zur Herstellung von Anthrachinonen der allgemeinen Formel I 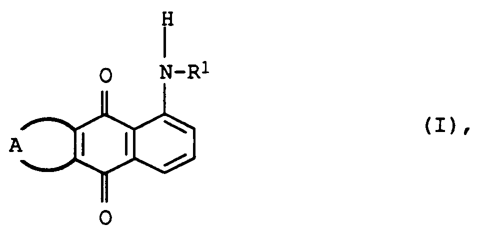 in der
R¹ Wasserstoff oder 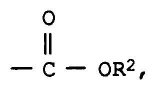
A 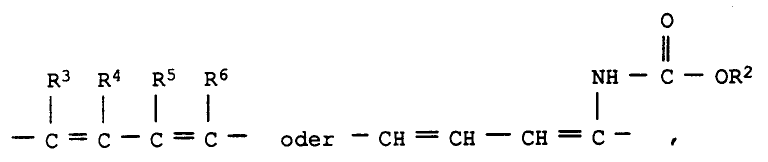
R² C₁- bis C₈-Alkyl, C₇- bis C₂₀-Phenylalkyl und
R³, R⁴, R⁵, R⁶ unabhängig voneinander Wasserstoff, C₁- bis C₈-Alkyl, C₇- bis C₂₀-Phenylalkyl, C₁- bis C₈-Alkoxy, C₁- bis C₈-Halogenalkyl, Nitro, Cyano, Halogen, Amino, C₁- bis C₈-Alkyl carbonyl, Benzoyl, N-C₁- bis C₈-Alkylphenylamino, C₁- bis C₈-Alkylsulfonyl, Phenylsulfonyl, Aminosulfonyl, Aminocarbonyl und Nitrobenzyl
bedeuten, dadurch gekennzeichnet, daß man N-Butadienylcarbamidsäureester der allgemeinen Formel II 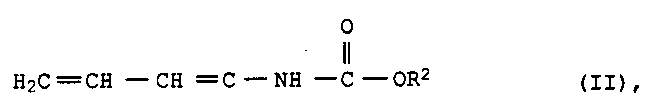 in der R² die obengenannten Bedeutungen hat,
a) für den Fall, daß A 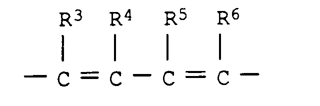 bedeutet, mit 1,4-Naphthochinonen der allgemeinen Formel III 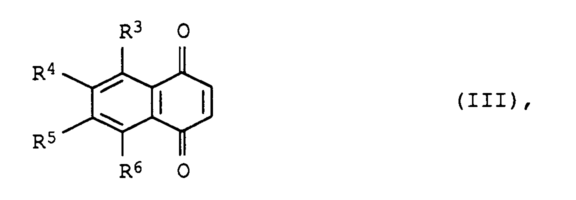 in der die Substituenten R³ bis R⁶ die obengenannten Bedeutungen haben, oder
b) für den Fall, daß A 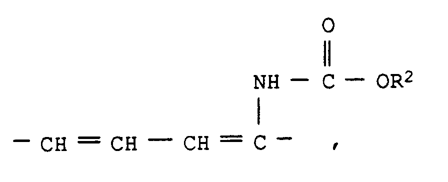 bedeutet, mit 1,4-Benzochinon
bei Temperaturen von 0 bis 150°C, und anschließend die entstandenen Carbamoylhydroanthrachinone in einem tertiären Amin mit sauerstoffhaltigen Gasen in Gegenwart von Kupfer-salzen umsetzt, und gegebenenfalls die entstandenen Carbamoylanthrachinone mit Hydroxidlösungen bei Temperaturen von 0 bis 150°C behandelt.

2. Verfahren zur Herstellung von Aminoanthrachinonen der allgemeinen Formel I nach Anspruch 1, dadurch gekennzeichnet, daß man N-Butadienylcarbamidsäureester II mit 1,4-Naphthochinonen III oder 1,4-Benzochinon bei Temperaturen von 0 bis 150°C in einem tertiären Amin mit sauerstoffhaltigen Gasen in Gegenwart von Kupfer-salzen umsetzt.

## Claims

1. A process for preparing anthraquinones of the general formula I 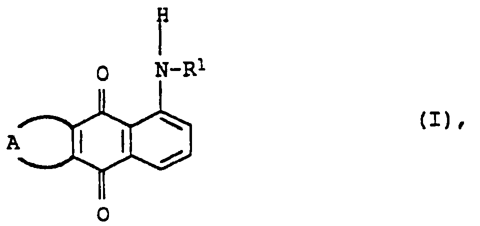 where
R¹ is hydrogen or 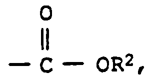
A is 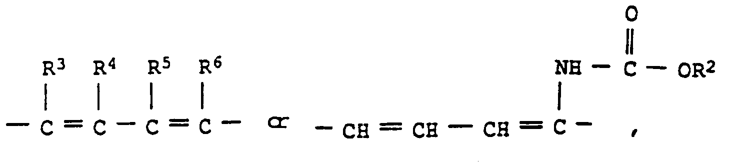
R² is C₁- to C₈-alkyl or C₇- to C₂₀-phenylalkyl and
R³, R⁴, R⁵ and R⁶, independently of one another, are hydrogen, C₁- to C₈-alkyl, C₇- to C₂₀-phenylalkyl, C₁- to C₈-alkoxy, C₁- to C₈-haloalkyl, nitro, cyano, halogen, amino, C₁- to C₈-alkylcarbonyl, benzoyl, N-C₁- to C₈-alkylphenylamino, C₁- to C₈-alkylsulfonyl, phenylsulfoyl, aminosulfonyl, aminocarbonyl and nitrobenzyl
which comprises reacting N-butadienylcarbamic acid esters of the general formula II 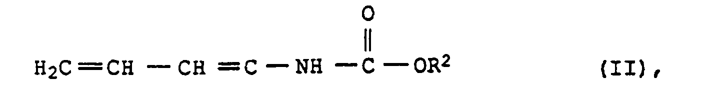 where R² has the abovementioned meanings,
a) in the case in which A is 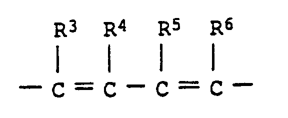 with 1,4-naphthoquinones of the general formula III 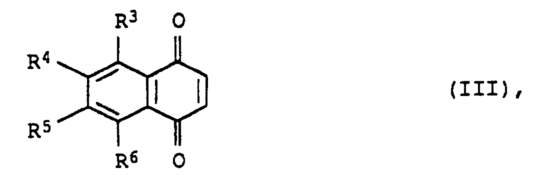 where the substituents R³ to R⁶ have the above-mentioned meanings, or
b) in the case in which A is 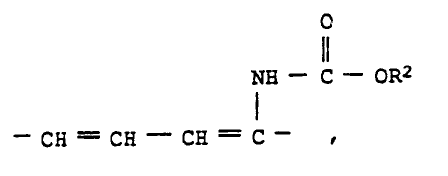 with 1,4-benzoquinone
at from 0 to 150°C, and then reacting the resulting carbamoylhydroanthraquinones with oxygen-containing gases in a tertiary amine in the presence of copper salts, and, if desired, treating the resulting carbamoylanthraquinones with hydroxide solutions at from 0 to 150°C.

2. A process for preparing aminoanthraquinones of the general formula I as claimed in claim 1, which comprises reacting N-butadienylcarbamic acid esters II with 1,4-naphthoquinones III or 1,4-benzoquinone with oxygen-containing gases in a tertiary amine at from 0 to 150°C in the presence of copper salts.

## Revendications

1. Procédé de préparation d'anthraquinones de la formule générale I 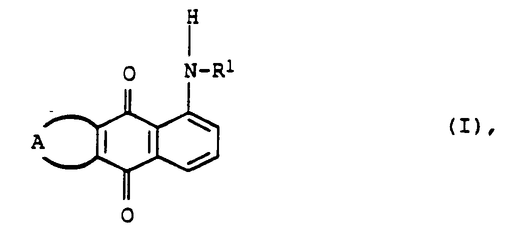 dans laquelle
R¹ représente un atome d'hydrogène ou le radical 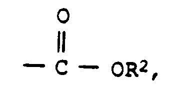
A représente le radical 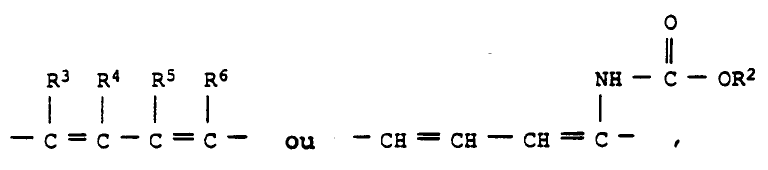
R² représente un radical alkyle en C₁ à C₈, phénylalkyle en C₇ à C₂₀ et
R³, R⁴, R⁵, R⁶ représentent, chacun indépendamment l'un de l'autre, un atome d'hydrogène, un radical alkyle en C₁ à C₈, phénylalkyle en C₇ à C₂₀, alcoxy en C₁ à C₈, halogénoalkyle en C₁ à C₈, nitro, cyano, un atome d'halogène, un radical amino, alkyl(C₁-C₈)carbonyle, benzoyle, N-alkyl(C₁-C₈)phénylamino, alkylsulfonyle en C₁ à C₈, phénylsulfonyle, aminosulfonyle, aminocarbonyle et nitrobenzyle,
caractérisé en ce que l'on fait réagir des esters de l'acide N-butadiénylcarbamique de la formule générale II 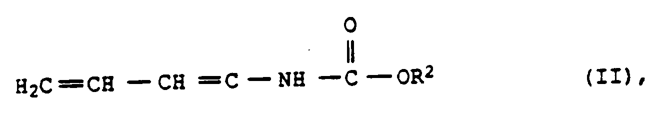 dans laquelle R² possède les significations qui lui ont été attribuées ci-dessus,
a) dans le cas où A représente un radical 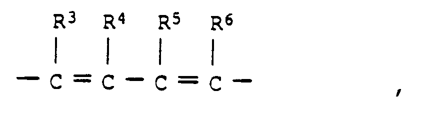 avec des 1,4-naphtoquinones de la formule générale III 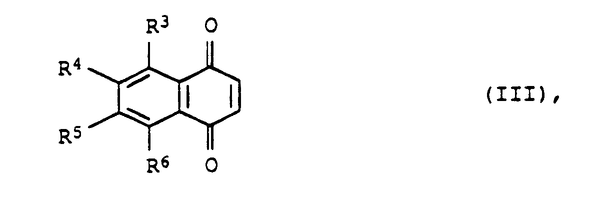 dans laquelle les substituants R³ à R⁶ possèdent les significations qui leur ont été précédemment attribuées, ou bien
b) dans le cas où A représente le radical 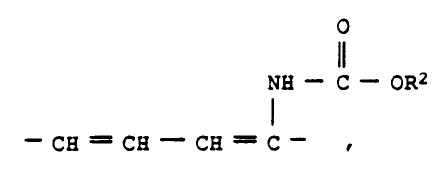 avec la 1,4-benzoquinone
à des températures de 0 à 150°C et on convertit ensuite les carbamoylhydroanthraquinones ainsi formées en une amine tertiaire avec des gaz contenant de l'oxygène et en présence de sels de cuivre et on traite éventuellement les carbamoylanthraquinones formées par des solutions d'hydroxydes à des températures de 0 à 150°C.

2. Procedé de préparation d'aminoanthraquinones de la formule générale I suivant la revendication 1, caractérisé en ce que l'on convertit les esters de l'acide N-butadiénylcarbamique II avec des 1,4-naphtoquinones III ou la 1,4-benzoquinone, à des températures de 0 à 150°C, en une amine tertiaire, avec des gaz contenant de l'oxygène et en présence de sels de cuivre.
